# EUROPEAN PATENT APPLICATION

(11) **EP 4 850 678 A1**
(43) Date of publication of application: **09.12.2026**
(21) Application number: 25748032.7
(22) Date of filing: 29.01.2025
(51) Int. Cl.: A61K 36/605, A61P 13/08

(54) **USE OF MORUS ALBA EXTRACT IN PREPARATION OF DRUG FOR TREATING AND/OR PREVENTING PROSTATE DISEASES**

(30) Priority: 04.02.2024 CN 202410162765; 26.01.2025 CN 202510125310
(71) Applicant: Beijing Wehand-Bio Pharmaceutical Co., Ltd, Beijing 102600 (CN); Guangxi Wehand-Bio Pharmaceutical Co., Ltd, Hechi, Guangxi 546300 (CN)
(72) Inventor: LIU, Yuling, Beijing 102600 (CN); LIU, Yuanyuan, Beijing 102600 (CN); MA, Luyao, Beijing 102600 (CN); HU, Wangmin, Beijing 102600 (CN); LI, Huijuan, Beijing 102600 (CN); LIU, Dongdong, Beijing 102600 (CN); FENG, Yu, Beijing 102600 (CN); LIU, Zhihua, Beijing 102600 (CN); ZHU, Xiangyang, Beijing 102600 (CN); WANG, Xingang, Beijing 102600 (CN); JIN, Yiqun, Beijing 102600 (CN); ZOU, Yuanyuan, Beijing 102600 (CN)
(74) Representative: Bandpay & Greuter
(86) International application number: PCT/CN2025/075608
(87) International publication number: WO 2025/162470

(57) **Abstract**

Use of a mulberry extract in preparing a drug for treating and/or preventing prostate diseases, wherein the mulberry extract contains alkaloids with a weight content of more than 3%, and/or contains polysaccharides with a weight content of no more than 70%, and/or contains flavonoids with a weight content of no more than 10%, and/or contains amino acids with a weight content of no more than 50%, and/or contains other components. The mulberry extract exhibits remarkable therapeutic effects on prostate-related diseases.

## Description

### TECHNICAL FIELD

The present disclosure relates to the field of biomedicine, specifically to the use of a mulberry extract in preparing a drug for treating and/or preventing prostatic diseases.

### BACKGROUND

The human prostate is a hormone-dependent organ. Starting approximately at the age of 40, the transitional zone of the prostate in male begins to grow slowly at an annual rate of 1.6%, and the growth accelerates with age.

Prostatic hyperplasia, also called as benign prostatic hyperplasia (BPH), is a urinary system disease prevalent among middle-aged and elderly males. It is mainly characterized histologically by hyperplasia of prostatic stromal and glandular components, anatomically by prostate enlargement, clinically by lower urinary tract symptoms, and urodynamically by bladder outlet obstruction. For males over 50 years old, more than 50% have an enlarged prostate volume, and the proportion rises to as high as 90% in males over 80 years old, showing that the incidence of BPH increasingly rises with population aging. Multiple factors contribute to the onset of BPH, including age, sex hormones, growth factors, and chronic inflammation. Sex hormone disturbance is the dominant factor leading to BPH. Prostate growth relies on the synergistic effects of estrogen and androgen, and an imbalance in the estrogen-androgen ratio is one cause of the occurrence and progression of BPH. Clinically applied drugs for BPH treatment include α-receptor blockers, 5α-reductase inhibitors, M-receptor antagonists, phosphodiesterase-5 inhibitors, and β₃-agonists and so on.

Prostatitis refers to prostatic diseases caused by multiple complex factors, with urethral irritation symptoms and chronic pelvic pain as the main clinical manifestations. As a common urological disease, prostatitis ranks first among male urological patients under 50 years old, with an overall prevalence rate of approximately 26.78% in China. According to the prostatitis classification criteria formulated by the National Institutes of Health (NIH) of the United States in 1995, prostatitis is divided into acute bacterial prostatitis (ABP), chronic bacterial prostatitis (CBP), chronic prostatitis (CP)/chronic pelvic pain syndrome (CPPS), and asymptomatic inflammatory prostatitis. Non-bacterial prostatitis is far more common than bacterial prostatitis. As the most prevalent clinical type of prostatitis, CP/CPPS accounts for over 90% of chronic prostatitis cases. Its main clinical manifestations include lower abdominal and perineal pain or pelvic discomfort, as well as lower urinary tract symptoms such as frequent micturition, urgent micturition, and dysuria. Some patients also suffer from psychological disorders including anxiety, depression and fear, and sexual dysfunctions such as erectile dysfunction and premature ejaculation, which severely impair patients' physical and mental health and quality of life, and increase socioeconomic burdens. Current clinical drugs for prostatitis treatment include α-receptor blockers, antibiotics, and non-steroidal anti-inflammatory drugs.

BPH and prostatitis seriously damage not only physical but also mental health of patients. Western medicines are mainly used for treatment, which primarily relieve symptoms but with numerous adverse reactions, lack of specificity, and unsatisfactory long-term therapeutic effects. Therefore, it is extremely necessary to develop drugs for prostatic diseases with low side effects and favorable prognosis.

### SUMMARY

The objective of the present disclosure is to provide use of a mulberry extract in preparing a drug for treating and/or preventing prostatic diseases. In another aspect, the present disclosure provides use of a mulberry extract for treating and/or preventing prostatic diseases. In a further aspect, the present disclosure provides a method for treating and/or preventing prostatic diseases, including administering a therapeutically or prophylactically effective amount of the mulberry extract to a patient in need thereof. The mulberry extract exhibits remarkable therapeutic effects on prostate-related diseases.

In the present disclosure, taking total weight percentage content of all components in the mulberry extract as 100%, the mulberry extract contains: alkaloids with a weight content of more than 3% (optionally contains alkaloids with a weight content ranging from 3% to 99%, further optionally contains alkaloids with a weight content ranging from 15% to 99%, further optionally contains alkaloids with a weight content ranging from 30% to 99%, further optionally contains alkaloids with a weight content ranging from 40% to 99%, further optionally contains alkaloids with a weight content ranging from 50% to 99%, and further optionally contains alkaloids with a weight content ranging from 60% to 99%),
and/or contains polysaccharides with a weight content of no more than 70% (optionally contains polysaccharides with a weight content ranging from 0.2% to 70%, further optionally contains polysaccharides with a weight content ranging from 0.2% to 50%, further optionally contains polysaccharides with a weight content ranging from 0.2% to 35%, further optionally contains polysaccharides with a weight content ranging from 0.2% to 25%, further optionally contains polysaccharides with a weight content ranging from 0.2% to 23%, and further optionally contains polysaccharides with a weight content ranging from 20% to 25%),
and/or contains flavonoids with a weight content of no more than 10% (optionally contains flavonoids with a weight content ranging from 0.05% to 5%, further optionally contains flavonoids with a weight content ranging from 0% to 2%, further optionally contains flavonoids with a weight content ranging from 0.05% to 2%, further optionally contains flavonoids with a weight content ranging from 0.5% to 1.5%, further optionally contains flavonoids with a weight content ranging from 0% to 1%, and further optionally contains flavonoids with a weight content ranging from 0.05% to 1%),
and/or contains amino acids with a weight content of no more than 50% (optionally contains amino acids with a weight content ranging from 0% to 30%, further optionally contains amino acids with a weight content ranging from 0% to 25%, further optionally contains amino acids with a weight content ranging from 0% to 20%, further optionally contains amino acids with a weight content ranging from 0% to 5%, further optionally contains amino acids with a weight content ranging from 3% to 25%, and further optionally contains amino acids with a weight content ranging from 5% to 20%),
and/or contains other components (optionally with a weight content ranging from 0% to 25%, further optionally with a weight content ranging from 0% to 20%, further optionally with a weight content ranging from 0% to 15%, further optionally with a weight content ranging from 0% to 11%, further optionally with a weight content ranging from 2% to 20%, and further optionally with a weight content ranging from 4% to 8%).

Optionally, the prostatic diseases include at least one of prostatitis and benign prostatic hyperplasia;
optionally, the prostatitis includes at least one of acute bacterial prostatitis, chronic bacterial prostatitis, chronic non-bacterial prostatitis, chronic pelvic pain syndrome, and asymptomatic inflammatory prostatitis;
optionally, symptoms of the chronic prostatitis include lower abdominal and perineal pain or pelvic discomfort, which may be accompanied by frequent micturition, urgent micturition, dysuria, urinary tract obstruction, sexual dysfunction, or depressive symptoms;
optionally, treating and/or preventing benign prostatic hyperplasia includes at least one of the following:
   1) reducing prostate-specific antigen (PSA) content;
   2) alleviating pathological damage to prostate tissues; and
   3) inhibiting proliferation of benign prostatic hyperplasia cells;
optionally, treating and/or preventing prostatitis includes at least one of the following:
   (1) alleviating pathological damage to patients' prostate tissues; and
   (2) reducing prostate-specific antigen (PSA) content.

In one embodiment, alleviating pathological damage to prostate tissues includes any one of the following: single-layer cuboidal epithelial cells in prostatic lumens, enlarged glandular lumens, relatively clear tissue structure, or reduced inflammatory cell infiltration in glandular stroma.

In one embodiment, the prostatic diseases are induced by high-fat diet and/or obesity.

In another aspect, the present disclosure provides use of a mulberry extract, including at least one of (a1) to (a3) as follows:
(a1) use in preparing a drug for reducing prostate-specific antigen (PSA) content;
(a2) use in preparing a drug for alleviating pathological damage to prostate tissues; and
(a3) use in preparing a drug for inhibiting proliferation of benign prostatic hyperplasia cells.

In another aspect, the present disclosure provides use of a mulberry extract, including at least one of (b1) to (b3) as follows:
(a1) use in reducing prostate-specific antigen (PSA) content;
(a2) use in alleviating pathological damage to prostate tissues; and
(a3) use in inhibiting proliferation of benign prostatic hyperplasia cells.

Optionally, taking total weight percentage content of all components in the mulberry extract as 100%, the weight content of each component in the mulberry extract is as follows:

| | |
|---|---|
| alkaloids | 3% to 99%, |
| polysaccharides | 0.2% to 70%, |
| flavonoids | 0% to 10%, |
| amino acids | 0% to 50%, and |
| other components | 0% to 25%; |

optionally, taking total weight percentage content of all components in the mulberry extract as 100%, the weight content of each component in the mulberry extract is as follows:

| | |
|---|---|
| alkaloids | 30% to 99%, |
| polysaccharides | 0.2% to 35%, |
| flavonoids | 0% to 2%, |
| amino acids | 0% to 30%, and |
| other components | 0% to 20%; |

optionally, taking total weight percentage content of all components in the mulberry extract as 100%, the weight content of each component in the mulberry extract is as follows:

| | |
|---|---|
| alkaloids | 30% to 99%, |
| polysaccharides | 0.2% to 35%, |
| flavonoids | 0.05% to 2%, |
| amino acids | 0% to 25%, and |
| other components | 0% to 20%; |

optionally, taking total weight percentage content of all components in the mulberry extract as 100%, the weight content of each component in the mulberry extract is as follows:

| | |
|---|---|
| alkaloids | 40% to 99%, |
| polysaccharides | 0.2% to 25%, |
| flavonoids | 0.05% to 1%, |
| amino acids | 0% to 25%, and |
| other components | 0% to 15%; |

optionally, taking total weight percentage content of all components in the mulberry extract as 100%, the weight content of each component in the mulberry extract is as follows:

| | |
|---|---|
| alkaloids | 50% to 99%, |
| polysaccharides | 0.2% to 23%, |
| flavonoids | 0% to 1%, |
| amino acids | 0% to 20%, and |
| other components | 0% to 11%; |

optionally, taking total weight percentage content of all components in the mulberry extract as 100%, the weight content of each component in the mulberry extract is as follows:

| | |
|---|---|
| alkaloids | 50% to 99%, |
| polysaccharides | 0.2% to 35%, |
| flavonoids | 0% to 2%, |
| amino acids | 0% to 30%, and |
| other components | 0% to 20%; |

optionally, taking total weight percentage content of all components in the mulberry extract as 100%, the weight content of each component in the mulberry extract is as follows:

| | |
|---|---|
| alkaloids | 60% to 99%, |
| polysaccharides | 0.2% to 23%, |
| flavonoids | 0% to 1%, |
| amino acids | 0% to 5%, and |
| other components | 0% to 11%; |

further optionally, taking total weight percentage content of all components in the mulberry extract as 100%, the weight content of each component in the mulberry extract is as follows:

| | |
|---|---|
| alkaloids | 50% to 65%, |
| polysaccharides | 20% to 25%, |
| flavonoids | 0.5% to 1.5%, |
| amino acids | 3% to 25%, and |
| other components | 2% to 20%; and |

further optionally, taking total weight percentage content of all components in the mulberry extract as 100%, the weight content of each component in the mulberry extract is as follows:

| | |
|---|---|
| alkaloids | 50% to 65%, |
| polysaccharides | 20% to 25%, |
| flavonoids | 0.5% to 1.5%, |
| amino acids | 5% to 20%, and |
| other components | 4% to 8%. |

Optionally, based on the mulberry extract, the weight content of each component is as follows:

| | |
|---|---|
| alkaloids | 15% to 99%, |
| polysaccharides | 0.2% to 20%, |
| flavonoids | 0% to 10%, |
| amino acids | 0% to 45%, and |
| other components | 0% to 15%; |

optionally, based on the mulberry extract, the weight content of each component is as follows:

| | |
|---|---|
| alkaloids | 15% to 90%, |
| polysaccharides | 3% to 20%, |
| flavonoids | 0% to 10%, |
| amino acids | 0% to 45%, and |
| other components | 0% to 15%; |

optionally, based on the mulberry extract, the weight content of each component is as follows:

| | |
|---|---|
| alkaloids | 15% to 80%, |
| polysaccharides | 5% to 35%, |
| flavonoids | 0% to 10%, |
| amino acids | 0% to 45%, and |
| other components | 0% to 15%; |

optionally, based on the mulberry extract, the weight content of each component is as follows:

| | |
|---|---|
| alkaloids | 15% to 70%, |
| polysaccharides | 0.2% to 20%, |
| flavonoids | 0% to 10%, |
| amino acids | 0% to 45%, and |
| other components | 0% to 15%; |

optionally, based on the mulberry extract, the weight content of each component is as follows:

| | |
|---|---|
| alkaloids | 15% to 70%, |
| polysaccharides | 0.2% to 20%, |
| flavonoids | 0% to 10%, |
| amino acids | 5% to 45%, and |
| other components | 0% to 15%; |

optionally, based on the mulberry extract, the weight content of each component is as follows:

| | |
|---|---|
| alkaloids | 15% to 65%, |
| polysaccharides | 0.2% to 20%, |
| flavonoids | 0% to 10%, |
| amino acids | 0% to 45%, and |
| other components | 0% to 15%; |

optionally, based on the mulberry extract, the weight content of each component is as follows:

| | |
|---|---|
| alkaloids | 15% to 65%, |
| polysaccharides | 0.2% to 20%, |
| flavonoids | 0% to 10%, |
| amino acids | 5% to 45%, and |
| other components | 0% to 15%; and |

in the mulberry extract, the weight content of each component is as follows:

| | |
|---|---|
| alkaloids | 15% to 30%, |
| polysaccharides | 0.2% to 40%, |
| flavonoids | 0% to 10%, |
| amino acids | 20% to 50%, and |
| other components | 0% to 15%. |

Further preferably, the alkaloids include one or more of 1-deoxynojirimycin (DNJ), N-methyl-1-deoxynojirimycin, fagomine (FAG), 3-epi-fagomine, 1,4-dideoxy-1,4-imino-D-arabinitol (DAB), calysteginB2, calysteginC1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol; and
optionally, weight percentage of DNJ is no less than 50% (optionally 60% to 99%) of total alkaloids.

Optionally, content of heavy metal in the mulberry extract is no more than 10 ppm.

Optionally, the mulberry extract acts on human beings or mammals.

Optionally, the drug is in an oral dosage form.

Optionally, the drug is prepared into tablets, capsules, oral solutions, oral emulsions, pills, granules, syrups and powder.

In the present disclosure, the mulberry extract may be provided in the form of commercially available Ramulus Mori Alkaloids Tablets (National Medicine Approval No. Z20200002).

Optionally, the mulberry extract of the present disclosure may be prepared according to the method disclosed in CN110393738A. In one embodiment, the preparation of the mulberry extract includes the following steps of: preparing a crude extraction solution of Moraceae plants; optionally, performing separation via a cation resin and/or an anion resin; optionally, subjecting resin effluent to alcohol precipitation and collecting the supernatant; and optionally, concentrating and/or drying the supernatant.

Optionally, the preparation of the mulberry extract in the present disclosure includes the following steps of: 1) preparing a crude extraction solution of Moraceae plants; 2) separating the crude extraction solution via a cation resin and/or an optional anion resin to afford a resin effluent; optional step 3): subjecting the resin effluent afforded in step 2) to alcohol precipitation and collecting the supernatant; and 4) concentrating and/or drying the supernatant. Optionally, the resin effluent afforded in step 2) is concentrated and/or dried before alcohol precipitation.

Optionally, the Moraceae plants are one or a combination of several of Morus multicaulis Perrott., Morus alba L., Morus atropurpurea Roxb., Morus mizuho Hotta, Morus wittiorum Hand Mazz., Morus laevigata Wall, Morus nigra Linn., Morus cathayana Hemsi., Morus serrata Roxb., Morus mongolica Schneid., Morus bombycis Koidz., Morus notabilis Schneid., Morus nigriformis Koidz., Morus yunnanensis Koidz., Morus australis Poir., Morus mongolica (Bur.) Schneid var. diabolica Koidz., large-leaf mulberry, Morus alba Var. Pendula Dippel, white-vein mulberry, mulberry varieties bred from the above mulberry species, and intraspecific or interspecific hybrid mulberry selected from the above mulberry species; and
optionally, the Moraceae plants are selected from one or more of Morus atropurpurea Roxb., Morus multicaulis Perrott., Morus alba L., Morus serrata Roxb., Morus bombycis Koidz. and hybrid mulberry; wherein the hybrid mulberry is optionally Yuesang No.11, Morus atropurpurea cv. Guisangyou 62 or Morus atropurpurea cv. Sangteyou 2. All parts of the Moraceae plants can be used, including leaves, roots, twigs, barks, buds, stems and fruits; optionally Ramulus Mori, Folium Mori or Cortex Mori can be used.

In the present disclosure, the mulberry extract may be Ramulus Mori extract, Cortex Mori extract, Folium Mori extract or a mixed extract thereof.

In one embodiment, the mulberry extract was prepared according to the following steps of: crushing Moraceae plants; heating for reflux extraction with water and/or alcohol solution or acidic water, the solvent dosage being 3 to 20 times of the raw medicinal material; extracting repeatedly for 1 to 3 times; combining and concentrating the extraction solutions; loading a cation exchange resin; eluting with 0.2 to 3 N ammonia water, loading the eluate onto an anion exchange resin, and collecting the non-adsorbed fraction; and adding ethanol to precipitate and remove impurities, and concentrating and/or drying, thereby affording the extract.

In one embodiment, the mulberry extract was prepared according to the following steps of: crushing Moraceae plants; heating for reflux extraction with water and/or alcohol solution or acidic water, wherein the solvent dosage was 3 to 20 times of the raw medicinal material; extracting repeatedly for 1 to 3 times; combining and concentrating the extraction solutions; loading a cation exchange resin; eluting with 0.2 to 3 N ammonia water, loading the eluate onto an anion exchange resin, and collecting the non-adsorbed substances; and concentrating and/or drying, thereby affording the extract.

In one embodiment, the mulberry extract was prepared according to the following steps of: crushing Moraceae plants; heating for reflux extraction with water and/or alcohol solution or acidic water, wherein the solvent dosage was 3 to 20 times of the raw medicinal material; extracting repeatedly for 1 to 3 times; combining and concentrating the extraction solutions; loading a cation exchange resin; eluting with 0.2 to 3 N ammonia water, and concentrating and/or drying the eluate, thereby affording the extract.

In one embodiment, the mulberry extract was prepared according to the following steps of: crushing Moraceae plants; heating for reflux extraction with water, wherein the solvent dosage was 3 to 20 times of the raw medicinal material (optionally 4 to 15 times, and further optionally 4 to 12 times); extracting repeatedly for 1 to 3 times (the extraction duration being optionally 0.5 to 3 h for each extraction, and further optionally 1 to 2 h for each extraction); combining and concentrating the extraction solutions; loading a cation exchange resin; eluting with 0.2 to 3 N ammonia water, loading the eluate onto an anion exchange resin, and collecting the non-adsorbed substances (i.e., anion resin effluent); and adding ethanol to precipitate and remove impurities, and concentrating and/or drying, thereby affording the extract.

Optionally, the concentrated crude extraction solution might be subjected to alcohol precipitation before resin separation in step 2). During alcohol precipitation, the crude extraction solution was added with ethanol, and then was stirred till the mixture was even; then stirring was stopped and the mixture was allowed to stand for a certain period of time so that insoluble substances therein precipitated. Optionally, calculated in L/kg, the volume-mass ratio of added ethanol to plant raw material was 0.2 to 20 times, optionally 0.4 to 10 times. Further optionally, an alcohol precipitation tank was used for alcohol precipitation. Optionally, the stirring speed during alcohol precipitation was 10 to 600 rpm, optionally 40 to 500 rpm, and further optionally 80 to 400 rpm or 300 rpm.

Optionally, after being packed into a column, the cation resin was activated by being washed with an acid solution, an alkaline solution, and an acid solution successively. Optionally, washing with an alkaline solution stopped until the pH of eluate reached 8.0 to 9.5, optionally 8.5 to 9.5; optionally, the alkaline solution was selected from ammonia solution, sodium hydroxide solution, potassium hydroxide solution or sodium carbonate solution; optionally, the concentration of the alkaline solution was 0.5 to 4 mol/L, optionally 1 to 2 mol/L. Optionally, the washing with an acid solution stopped until the pH of eluate reached 3.0 to 7.0, optionally 4.5 to 6.5. Optionally, the acid solution was selected from hydrochloric acid solution, phosphoric acid solution, disodium hydrogen phosphate-citric buffer; optionally, the concentration of the acid solution was 0.5 to 4 mol/L, optionally 1.5 to 2 mol/L. Optionally, after the last washing with an acid solution, the cation resin was rinsed with deionized water having a volume of 3 to 5 times the column volume.

Optionally, the cation resin is one or a combination of more selected from 732-type strongly acidic styrene-based cation exchange resin, 002SC-type strongly acidic styrene-based cation resin, 734-type strongly acidic styrene-based cation exchange resin, D001-type macroporous strongly acidic styrene-based cation exchange resin, D113-type macroporous and weakly acidic cation resin and D254-type macroporous and strongly alkaline quaternary ammonium-type cation exchange resin.

Optionally, the amount of the cation resin used and the charged plant raw material had a weight ratio ranging from 1:1 to 1:30 (optionally 1:1 to 1:25, 1:2 to 1:20, 1:2 to 1:15, 1:2 to 1:10, 1:2 to 1:7, 1:2 to 1:3).

After loading the plant crude extraction solution onto the cation resin, the loaded cation resin was eluted with an eluent. Optionally, the eluent was a salt solution or an alkaline solution containing cations, optionally one or more selected from sodium chloride, ammonium chloride, ammonium sulfate, ammonium nitrate, ammonia water, potassium chloride and sodium hydroxide.

Optionally, the eluent had a concentration ranging from 0.04 to 5 mol/L (optionally 0.5 to 2.5 mol/L, 0.2 to 3 mol/L, and further optionally 0.5 to 2.5 mol/L).

Optionally, the eluent had a flow rate ranging from 1 to 15 BV/h (optionally 5 to 10 BV/h, and further optionally 5 to 6 BV/h).

Optionally, the weight of the eluent used for the separation via cation resin was 0.1 to 30 times the weight of the charged plant raw material, optionally, the elution was performed with the eluent, of which the weight was 0.5 to 10 times the weight of the charged plant raw material.

The starting point of collection may be determined according to the pH of the cation resin effluent; for example, when an alkaline solution such as ammonia water was used for elution, the eluate was collected when the pH of the cation column effluent was detected to be higher than 7, or the collection starting point of the effluent was determined according to a chromogenic reaction or a precipitation reaction; optionally, the collection was terminated when the volume of the collection solution reached 0.1 to 10 times the weight of the charged plant raw material (further optionally 0.2 to 5 times), and the collection solution was optionally purified by passing through an anion column.

For purification via anion column, optionally, after being packed into the column, the anion resin was activated by being washed with an alkaline solution, an acid solution and an alkaline solution successively.

Optionally, the anion resin was washed with an alkaline solution until the pH of the eluate reached 8.0 to 9.5, optionally 8.5 to 9.5.

Optionally, the alkaline solution was selected from ammonia solution, sodium hydroxide solution, potassium hydroxide solution or sodium carbonate solution; optionally, the concentration of the alkaline solution was 0.5 to 4 mol/L, optionally 1 to 2 mol/L.

Optionally, the anion resin was washed with an acid solution until the pH of the eluate reached 3.0 to 7.0, optionally 4.5 to 6.5. Optionally, the acid solution was selected from hydrochloric acid solution, phosphoric acid solution and disodium hydrogen phosphate-citric buffer; optionally, the concentration of the acid solution is 0.5 to 4 mol/L, optionally 1 to 2 mol/L.

Optionally, the anion resin was one or a combination of more selected from 711-type strongly alkaline styrene-based anion exchange resin, 717-type strongly alkaline styrene-based anion exchange resin, D201-type macroporous and strongly alkaline styrene-based anion exchange resin or D218-type macroporous and strongly alkaline acrylic-based anion exchange resin, D301-G-type macroporous and weakly acidic styrene-based anion exchange resin and D301-type macroporous and weakly alkaline styrene-based anion exchange resin.

Optionally, the amount of the anion resin used and the charged plant raw material had a weight ratio ranging from 1:1 to 1:80 (optionally 1:1 to 1:64, 1:1 to 1:32, 1:1 to 1:24, 1:5 to 1:16, 1:3).

Collection started when liquid flowed out of the anion resin. Optionally, the collection was terminated when the volume of the collected solution reached 0.05 to 10 times the weight of the charged plant raw material (optionally 0.1 to 5 times).

Optionally, the ethanol used for alcohol precipitation and the charged plant raw material had a weight ratio ranging from 1:4 to 1:600 (optionally 1:20 to 1:300, further optionally 1:20 to 1:50, 1:40, 1:80, 1:22). The stirring speed during alcohol precipitation was 10 to 600 rpm (optionally 40 to 500 rpm, 80 to 400 rpm). The alcohol precipitation duration was 12 to 24 h.

Further, prior to alcohol precipitation, the method further includes a step of concentrating by reverse osmosis membrane after impurities were removed from the anion resin effluent by conducting centrifugation or filtration via micro-filtration membrane. The specific gravity of the concentrated liquid might be 1.0 to 1.3, optionally 1.1 to 1.25.

Optionally, the drug further comprises a pharmaceutically acceptable carrier. The carrier is an inactive component that conforms to the administration route or the mode of administration and has no toxic effect on the human body. The carrier may be a solid or liquid excipient. Solid excipients, for example, include microcrystalline cellulose, mannitol, lactose, pregelatinized starch, low-substituted hydroxypropyl cellulose, crospovidone, sodium starch glycolate, aspartame, calcium hydrogen phosphate, sodium lactate, poloxamer, sodium dodecyl sulfate, sodium carboxymethyl cellulose, gelatin, xanthan gum, povidone, starch, magnesium stearate and talc; liquid excipients, for example, include water, ethanol, syrup and glycerol.

In the present disclosure, the mulberry extract is abbreviated as SZ-A.

### Beneficial Effects

The mulberry extract of the present disclosure can reduce PSA in obese mice, alleviate pathological damage to prostate tissues, improve prostatic tissue morphology, and lower inflammatory level. In a word, the mulberry extract can ameliorate prostate-related diseases including benign prostatic hyperplasia and prostatitis.

### Description of Drawings

Fig. 1 shows the effect of a mulberry extract on serum PSA in HFD-induced C57BL/6 obese mouse model in Test Example 1.
Fig. 2 shows the pathological effects of mulberry extract on prostate tissues of HFD-induced C57BL/6 obese mouse model in Test Example 1.
Fig. 3 is a bar chart showing BPH-1 cell viability in Groups A1 to A4 in Test Example 2.
Fig. 4 is a bar chart showing BPH-1 cell viability in Groups B1 to B4 in Test Example 2.

### Embodiments

### Preparation Example 1 of Mulberry Extract

1000 kg of fresh Ramulus Mori (Morus serrata Roxb-Yuesang No. 11) was taken and crushed, then added with 4000 L of water and extracted by heating reflux for 2 h; extraction solution was combined, and filtered to remove insoluble substances, and a crude extraction solution is thus afforded. The crude extraction solution was concentrated by heating until a mass percentage content of solid substances was up to 4%, and was kept at 50°C and served as a loading solution for a cation resin column.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 920 L.

The collection solution was centrifuged to remove impurities, and then was concentrated by means of a reverse osmosis membrane; a specific gravity of concentrated liquid was 1.25; the concentrated liquid was transferred to an alcohol precipitation tank, and 25 L of anhydrous ethanol was added when the stirring paddle was at 500 rpm. After the ethanol was added, stirring was stopped, and alcohol precipitation was performed for 24 h; the supernatant was taken and concentrated under reduced pressure to afford extractum of Ramulus Mori extract (i.e. the mulberry extract SZ-A).

In the extractum of Ramulus Mori extract, alkaloids had a mass percentage content of 52%, polysaccharides had a mass percentage content of 22%, flavonoids had a mass percentage content of 0.8%, and amino acids had a mass percentage content of 20%. Among the alkaloids, 1-DNJ had a content of 60%, FAG had a content of 17%, and DAB had a content of 15%.

### Preparation Example 2 of Mulberry Extract

10 kg of fresh Ramulus Mori (Sangteyou No. 2) were taken and crushed, then were added with 150 L of water in 2 times, and were extracted by means of decocting for 3 h each time; extraction solution was combined, and was filtered to remove insoluble substances. The extraction solution was concentrated by heating until a mass percentage content of solid substances was up to 8%, then was transferred to an alcohol precipitation tank, and was added with 2367.9 g (3 L) of anhydrous ethanol when the stirring paddle was at 300 rpm. After the ethanol was added, stirring was stopped, and alcohol precipitation was performed for 24 h; the supernatant was taken as a loading solution for the cation resin column. 5 kg of 002SC-type strongly acidic styrene-based cation resin was used for column packing, and the cation resin was activated according to the method described in Preparation Example 1. The extraction solution was loaded after concentration and alcohol precipitation, and then was eluted with 100 L of 5 mol/L potassium chloride at an elution speed of 5 BV/h; the effluent was detected with 20% silicotungstic acid, and collection was started upon generation of white precipitate; the collection was stopped until the collection solution was up to 25 L; and the collection solution was purified directly through the anion resin column.

10 kg of 711-type strongly alkaline styrene-based anion resin was used for column packing, and the anion resin was activated according to the method described in Preparation Example 1. The collected cation resin eluent was loaded onto the anion resin, and the effluent was collected until the effluent was up to 15 L. The collection solution was reloaded onto the cation resin, and was separated twice by using the cation resin and the anion resin in sequence according to the aforementioned method.

The collection solution afforded after three column separations was centrifuged to remove impurities, and then was concentrated through a reverse osmosis membrane, wherein the specific gravity of the concentrated liquid was 1.25; it was transferred to an alcohol precipitation tank, and was added with 125 g of anhydrous ethanol when the stirring paddle was at 1000 rpm. After the ethanol was added, the stirring was stopped, and alcohol precipitation was performed for 24 h; the supernatant was taken and concentrated under reduced pressure to afford extractum of extract. In addition, fresh Cortex Mori and Folium Mori (Sangteyou No. 2) were taken again and were extracted, wherein the extraction method and parameters were the same as those described above.

In the afforded extract of Ramulus Mori, alkaloids had a mass percentage content of 98%, polysaccharides had a mass percentage content of 0.2%, flavonoids had a mass percentage content of 0.05%, and amino acids had a mass percentage content of 0%. Among alkaloids, 1-DNJ had a content of 99%, FAG had a content of 0.5%, and DAB had a content of 0.4%.

In the afforded Cortex Mori extract, alkaloids had a mass percentage content of 95%, polysaccharides had a mass percentage content of 2%, flavonoids had a mass percentage content of 0.1%, and amino acids had a mass percentage content of 1%. Among the alkaloids, 1-DNJ had a content of 96%, FAG had a content of 1.5%, and DAB had a content of 1.4%.

In the afforded Folium Mori extract, alkaloids had a mass percentage content of 90%, polysaccharides had a mass percentage content of 4%, flavonoids had a mass percentage content of 0.1%, and amino acids had a mass percentage content of 3%. Among the alkaloids, 1-DNJ had a content of 91%, FAG had a content of 3.1%, and DAB had a content of 2.8%.

### Preparation Example 3 of Mulberry Extract

1000 kg of fresh Ramulus Mori (Morus atropurpurea Roxb) was taken and crushed, then was added with 11500 L of water and was extracted by heating reflux for 2 h; extraction solution was combined, and was filtered to remove insoluble substances, and a crude extraction solution is thus afforded. The crude extraction solution was centrifuged to remove impurities, and then was concentrated by means of a reverse osmosis membrane until a percentage of solid substances was up to 1%, and served as a loading solution for a cation resin column.

300 kg of D001-type macroporous and strongly acidic styrene-based cation resin was used for column packing, and the cation resin was activated according to the method described in Preparation 1. The crude extraction solution was loaded after concentration, and then was eluted with 5000 L of 0.04 mol/L ammonium nitrate at an elution speed of 5 BV/h; the effluent was detected with 20% silicotungstic acid, and collection was started upon generation of white precipitate; the collection was stopped until the collection solution was up to 1000 L.

The collection solution afforded after separation of cation column was concentrated by nanofiltration, and then was concentrated under reduced pressure to afford extractum of extract.

In the afforded Ramulus Mori extract, alkaloids had a mass percentage content of 15%, polysaccharides had a mass percentage content of 20%, flavonoids had a mass percentage content of 7%, and amino acids had a mass percentage content of 45%. Among the alkaloids, 1-DNJ had a content of 55%, FAG had a content of 23%, and DAB had a content of 10%.

### Preparation Example 4 of Mulberry Extract

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

125 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was higher than 8, was collected until the effluent was up to 870 L.

The collection solution afforded after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to afford extractum of Ramulus Mori extract. Contents in the sample: alkaloids had a mass percentage content of 80%, polysaccharides had a mass percentage content of 5%, flavonoids had a mass percentage content of 0.1%, and amino acids had a mass percentage content of 4%. Among the alkaloids, 1-DNJ had a content of 75%, FAG had a content of 12%, and DAB had a content of 10%.

### Preparation Example 5 of Mulberry Extract

400 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected extract was concentrated and loaded onto the anion resin, and the effluent was collected.

The collection solution afforded after anion column separation was filtered by means of micro-filtration membrane to remove impurities, then was concentrated through a reverse osmosis membrane, and was further concentrated under reduced pressure and dried to afford extractum of Ramulus Mori extract. Contents in the sample: alkaloids had a mass percentage content of 3%, polysaccharides had a mass percentage content of 70%, flavonoids had a mass percentage content of 10%, and amino acids had a mass percentage content of 10%. Among the alkaloids, 1-DNJ had a content of 68%, FAG had a content of 17%, and DAB had a content of 8%.

### Preparation Example 6 of Mulberry Extract

1500 kg of fresh Ramulus Mori (Morus serrata Roxb-Yuesang No. 11) was taken and crushed, then was added with 6000 L of water and was extracted by heating reflux for 2 h; extraction solution was combined, and was filtered to remove insoluble substances, and a crude extraction solution is thus afforded. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to afford extractum of Ramulus Mori extract, wherein: alkaloids had a mass percentage content of 30%, polysaccharides had a mass percentage content of 35%, flavonoids had a mass percentage content of 2%, and amino acids had a mass percentage content of 25%. Among the alkaloids, 1-DNJ had a content of 62%, FAG had a content of 20%, and DAB had a content of 13%.

### Preparation Example 7 of Mulberry Extract

1000 kg of fresh Ramulus Mori (Morus serrata Roxb-Yuesang No. 11) was taken and crushed, then was added with 4000 L of water and was extracted by heating reflux for 2 h; extraction solution was combined, and was filtered to remove insoluble substances, and a crude extraction solution is thus afforded. The crude extraction solution was concentrated by heating until a percentage of solid substances was up to 4%, and was kept at 50°C and served as a loading solution for a cation resin column.

100 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L. The effluent was concentrated under reduced pressure so as to afford extractum of Ramulus Mori extract, wherein: alkaloids had a mass percentage content of 40%, polysaccharides had a mass percentage content of 25%, flavonoids had a mass percentage content of 0.5%, and amino acids had a mass percentage content of 25%. Among the alkaloids, 1-DNJ had a content of 57%, FAG had a content of 24%, and DAB had a content of 16%.

### Preparation Example 8 of Mulberry Extract

333 kg of dried Ramulus Mori (Yuesang No. 11) was taken and crushed, then was added with 4000 L of water and was extracted twice by heating reflux, 1 h for each reflux; extraction solution was combined and filtered, and was concentrated until the crude drug quantity reached 1 kg/L.

150 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

62.5 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent, of which pH was higher than 8, was collected until the effluent was up to 870 L.

The collection solution afforded after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 400 rpm. After finishing adding the ethanol, the stirring was stopped, and alcohol precipitation was performed for 24 h; the supernatant was taken and concentrated under reduced pressure to afford extractum of Ramulus Mori extract. Contents in sample: alkaloids had a mass percentage content of 63%, polysaccharides had a mass percentage content of 23%, flavonoids had a mass percentage content of 1%, and amino acids had a mass percentage content of 5%. Among the alkaloids, 1-DNJ had a content of 61.9%, FAG had a content of 16.6%, and DAB had a content of 11.1%.

### Preparation Example 9 of Mulberry Extract

1000 kg of fresh Ramulus Mori (Yuesang No. 11) was taken and crushed, then was added with 4000 L of water and was extracted by heating reflux for 2 h; extraction solution was combined, and was filtered to remove insoluble substances, and a crude extraction solution is thus afforded. The crude extraction solution was concentrated by heating until a percentage of solid substances was up to 4%, and was kept at 50°C and served as a loading solution for a cation resin column.

120 kg of D113-type macroporous and weakly acidic phenylpropene-based cation resin was used for column packing, and a hydrochloric acid solution of 2 mol/L was used for washing until the pH of an eluent was 4.5; a sodium hydroxide solution of 1 mol/L was used for washing until the pH of an eluent was 8.5; a hydrochloric acid solution of 2 mol/L was used for washing until the pH of an eluent was 4.5; and deionized water, of which the volume was 5 times as that of the column, was used for rinsing, and activation was thus completed. The concentrated extraction solution was loaded, and then was eluted with 1000 L of 2.5 mol/L ammonia water at an elution speed of 6 BV/h; the eluent was collected when pH of the effluent from the cation column was detected to be higher than 7; collection was stopped when the collection solution was up to 900 L, and the collection solution was purified directly through the anion column.

45 kg of D218-type macroporous and strongly alkaline acrylic-based anion resin was used for column packing, and a sodium hydroxide solution of 1.5 mol/L was used for washing until the pH of the eluent was 9.0; a hydrochloric acid solution of 1.5 mol/L was used for washing until the pH of the eluent was 3.5; and a sodium hydroxide solution of 1.5 mol/L was used for washing until the pH of the eluent was 9.0, and the activation was thus completed. The collected cation resin eluent was loaded onto the anion resin, and the effluent was collected until the effluent was up to 870 L.

The collection solution afforded after anion column separation was filtered by means of micro-filtration membrane to remove impurities, and then was concentrated through a reverse osmosis membrane, wherein the specific gravity of the concentrated liquid was 1.1; it was transferred to an alcohol precipitation tank, and was added with 15 kg of anhydrous ethanol when the stirring paddle was at 300 rpm. After finishing adding the ethanol, the stirring was stopped, and alcohol precipitation was performed for 24 h; the supernatant was taken and concentrated under reduced pressure to afford extractum of the extract. Contents in sample: alkaloids had a mass percentage content of 70%, polysaccharides had a mass percentage content of 20%, flavonoids had a mass percentage content of 0.6%, and amino acids had a mass percentage content of 5%. Among the alkaloids, 1-DNJ had a content of 70%, FAG had a content of 13%, and DAB had a content of 10%.

### II. Efficacy Tests of Mulberry Extract

### Test Example 1: HFD-Induced C57BL/6 Obese Mouse Model

Forty-five healthy 6-week-old male C57BL/6 mice were randomly divided into a normal group (Chow), a high-fat diet model group (HFD), and a mulberry extract group (SZ-A), with 9 mice in each group (specific test grouping and administration dosages were shown in Table 1 below). The mulberry extract was the Ramulus Mori extract afforded in Preparation Example 9. Mice in the normal group were fed with basal diet, while those in the model group and in the SZ-A group were fed with high-fat diet (Research Diet, D12492, 60 kcal% Fat). After 8 weeks of feeding, each test group was given corresponding dosage of test drug for continuous 6 weeks, with high-fat diet maintained during treatment. Animal body weights, food intake and water intake were monitored simultaneously, and the model group was given normal saline. Samples were collected after the test for efficacy evaluation.

**Table 1 Test Grouping and Administration**

| Groups | | Administration dosages (calculated based on alkaloids) |
|---|---|---|
| Normal Group | Blank | 0 |
| Model Group | Model | 0 |
| SZ-A Group | SZ-A | 400 mg/kg/d |

### Detection indicators:

1) Serum prostate-specific antigen (PSA) content was detected using an ELISA kit.
2) Prostate tissues were collected for HE staining, and tissue morphology was observed under a microscope.

### Test Results:

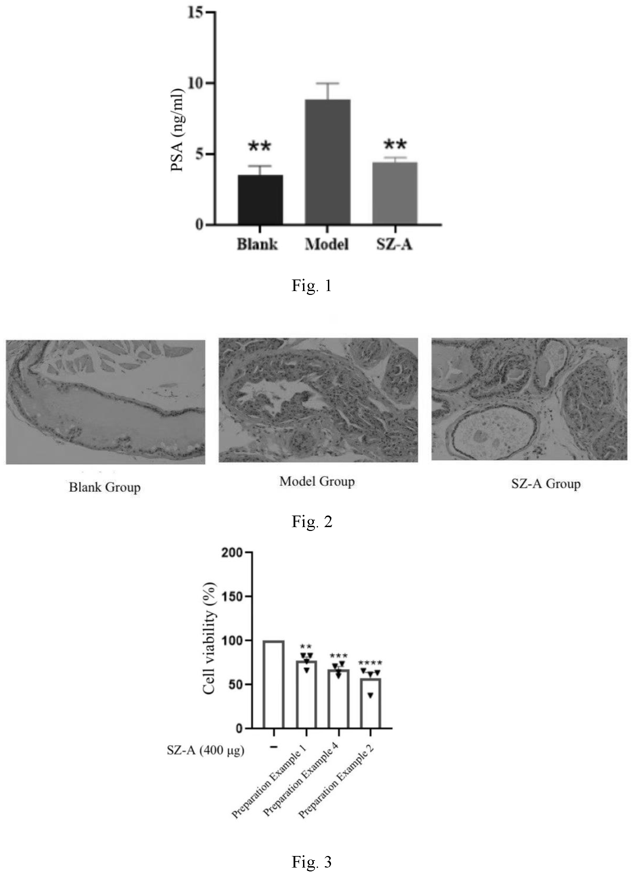

Fig. 1 shows that serum PSA content for mice in the Model Group was significantly increased compared with the Normal Group, indicating prostatic tissue lesions. Compared with the Model Group, SZ-A significantly reduced serum PSA content in mice. PSA is a single-chain glycoprotein secreted by prostatic epithelial cells, belonging to the kallikrein family; it exists in prostate tissues and semen with extremely low levels in normal human serum.

According to Fig. 2, prostatic glandular epithelial cells in the Blank Group were single-layer cuboidal with occasional finger-like protrusions, distinct glandular gaps, clear tissue structure and no abnormalities; regular acini gradually thinned into low cuboidal cells arranged in a uniform single layer. In the Model Group, significant hyperplasia of glandular epithelial cells in prostatic lumens was observed; epithelial cells lost polarity and protruded into glandular lumens in stratified or pseudo stratified structures, clustered in lumens with adjacent distribution, forming sieve-like lumens with significantly narrowed glandular cavities; glandular walls were markedly thickened, acinar density increased, gaps significantly narrowed, and some adjacent glandular lumens were distributed back-to-back; inflammatory cells were observed in glandular stroma with local fibrous tissue hyperplasia. Prostatic tissue lesions in the SZ-A Group were significantly improved, manifested as single-layer cuboidal epithelial cells in partial prostatic lumens, enlarged glandular lumens, relatively clear tissue structure, and markedly reduced inflammatory cell infiltration in glandular stroma.

According to the aforementioned results, SZ-A significantly reduced serum PSA content and alleviated prostatic tissue lesions, indicating that SZ-A had a certain ameliorative effect on both benign prostatic hyperplasia and prostatitis.

### Test Example 2: Effect of SZ-A on Human Benign Prostatic Hyperplasia Cell Model

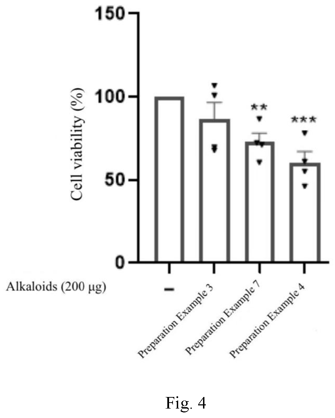

Human benign prostatic hyperplasia cells (BPH-1) were cultured in BPH-1-specific medium (RPMI-1640+20% FBS+20 ng/mL testosterone+0.5% ITS-G (100×)+1% P/S); cells were digested with 0.25% trypsin and collected, then seeded into 96-well plates at a density of 3 × 10³ cells per well with six replicates per group; after 24 h of seeding, when it was observed that the cell adherence confluence reached approximately 50%, cells were washed twice with PBS; then 100 µL of complete medium containing different concentrations of SZ-A was added according to the following groups, with normal saline replacing SZ-A as the Control Group. After 24 h of treatment, 10 µL of CCK-8 solution was added under light-shielded conditions. After another 2 h, the absorbance was measured using a microplate reader (at 450 nm wavelength after continuous shaking for 15 s). Cell viability = [(OD value of Administration Group - OD value of Blank Control Group) / (OD value of Control Group - OD value of Blank Control Group)] × 100%. Cell viability was detected via the CCK-8 method. The viability results of BPH-1 cells in Groups A1 to A4 were shown in Fig. 3, and those in Groups B1 to B4 were shown in Fig. 4.

### Grouping and Administration:

Group A1: Control Group;
Group A2: Administration Group, 400 µg/mL SZ-A (calculated by the extract; SZ-A being the Ramulus Mori extract from Preparation Example 1);
Group A3: Administration Group, 400 µg/mL SZ-A (calculated by the extract; SZ-A being the Ramulus Mori extract from Preparation Example 2);
Group A4:Administration Group, 400 µg/mL SZ-A (calculated by the extract; SZ-A being the Ramulus Mori extract from Preparation Example 4);
Group B1: Control group;
Group B2: Administration Group, 200 µg/mL SZ-A (calculated by alkaloids; SZ-A being the Ramulus Mori extract from Preparation Example 3);
Group B3: Administration Group, 200 µg/mL SZ-A (calculated by alkaloids; SZ-A being the Ramulus Mori extract from Preparation Example 7);
Group B4:Administration Group, 200 µg/mL SZ-A (calculated by alkaloids; SZ-A being the Ramulus Mori extract from Preparation Example 4);

The test results in Figs. 3 and 4 indicate that SZ-A can inhibit the proliferation of BPH-1 cells.

## Claims

1. Use of a mulberry extract in preparing a drug for treating and/or preventing prostatic diseases, wherein: taking total weight percentage content of all components in the mulberry extract as 100%, the mulberry extract contains: alkaloids with a weight content of more than 3% (optionally contains alkaloids with a weight content ranging from 3% to 99%, further optionally contains alkaloids with a weight content ranging from 15% to 99%, further optionally contains alkaloids with a weight content ranging from 30% to 99%, further optionally contains alkaloids with a weight content ranging from 40% to 99%, further optionally contains alkaloids with a weight content ranging from 50% to 99%, and further optionally contains alkaloids with a weight content ranging from 60% to 99%),
and/or contains polysaccharides with a weight content of no more than 70% (optionally contains polysaccharides with a weight content ranging from 0.2% to 70%, further optionally contains polysaccharides with a weight content ranging from 0.2% to 50%, further optionally contains polysaccharides with a weight content ranging from 0.2% to 35%, further optionally contains polysaccharides with a weight content ranging from 0.2% to 25%, further optionally contains polysaccharides with a weight content ranging from 0.2% to 23%, and further optionally contains polysaccharides with a weight content ranging from 20% to 25%),
and/or contains flavonoids with a weight content of no more than 10% (optionally contains flavonoids with a weight content ranging from 0.05% to 5%, further optionally contains flavonoids with a weight content ranging from 0% to 2%, further optionally contains flavonoids with a weight content ranging from 0.05% to 2%, further optionally contains flavonoids with a weight content ranging from 0.5% to 1.5%, further optionally contains flavonoids with a weight content ranging from 0% to 1%, and further optionally contains flavonoids with a weight content ranging from 0.05% to 1%),
and/or contains amino acids with a weight content of no more than 50% (optionally contains amino acids with a weight content ranging from 0% to 30%, further optionally contains amino acids with a weight content ranging from 0% to 25%, further optionally contains amino acids with a weight content ranging from 0% to 20%, further optionally contains amino acids with a weight content ranging from 0% to 5%, further optionally contains amino acids with a weight content ranging from 3% to 25%, and further optionally contains amino acids with a weight content ranging from 5% to 20%),
and/or contains other components (optionally with a weight content ranging from 0% to 25%, further optionally with a weight content ranging from 0% to 20%, further optionally with a weight content ranging from 0% to 15%, further optionally with a weight content ranging from 0% to 11%, further optionally with a weight content ranging from 2% to 20%, and further optionally with a weight content ranging from 4% to 8%).

2. The use according to claim 1, wherein: the prostatic diseases include at least one of prostatitis and benign prostatic hyperplasia;
optionally, the prostatitis includes at least one of acute bacterial prostatitis, chronic bacterial prostatitis, chronic non-bacterial prostatitis, chronic pelvic pain syndrome, and asymptomatic inflammatory prostatitis.

3. The use according to claim 1 or 2, wherein: treating and/or preventing benign prostatic hyperplasia includes at least one of the following:
1) reducing prostate-specific antigen (PSA) content;
2) alleviating pathological damage to prostate tissues; and
3) inhibiting proliferation of benign prostatic hyperplasia cells.

4. The use according to any one of claims 1 to 3, wherein: treating and/or preventing prostatitis includes at least one of the following:
(1) alleviating pathological damage to patients' prostate tissues; and
(2) reducing prostate-specific antigen (PSA) content.

5. Use of a mulberry extract, including at least one of (a1) to (a3) as follows:
(a1) use in preparing a drug for reducing prostate-specific antigen (PSA) content;
(a2) use in preparing a drug for alleviating pathological damage to prostate tissues; and
(a3) use in preparing a drug for inhibiting proliferation of benign prostatic hyperplasia cells;
wherein:taking total weight percentage content of all components in the mulberry extract as 100%, the mulberry extract contains: alkaloids with a weight content of more than 3% (optionally contains alkaloids with a weight content ranging from 3% to 99%, further optionally contains alkaloids with a weight content ranging from 15% to 99%, further optionally contains alkaloids with a weight content ranging from 30% to 99%, further optionally contains alkaloids with a weight content ranging from 40% to 99%, further optionally contains alkaloids with a weight content ranging from 50% to 99%, and further optionally contains alkaloids with a weight content ranging from 60% to 99%),
and/or contains polysaccharides with a weight content of no more than 70% (optionally contains polysaccharides with a weight content ranging from 0.2% to 70%, further optionally contains polysaccharides with a weight content ranging from 0.2% to 50%, further optionally contains polysaccharides with a weight content ranging from 0.2% to 35%, further optionally contains polysaccharides with a weight content ranging from 0.2% to 25%, further optionally contains polysaccharides with a weight content ranging from 0.2% to 23%, and further optionally contains polysaccharides with a weight content ranging from 20% to 25%),
and/or contains flavonoids with a weight content of no more than 10% (optionally contains flavonoids with a weight content ranging from 0.05% to 5%, further optionally contains flavonoids with a weight content ranging from 0% to 2%, further optionally contains flavonoids with a weight content ranging from 0.05% to 2%, further optionally contains flavonoids with a weight content ranging from 0.5% to 1.5%, further optionally contains flavonoids with a weight content ranging from 0% to 1%, and further optionally contains flavonoids with a weight content ranging from 0.05% to 1%),
and/or contains amino acids with a weight content of no more than 50% (optionally contains amino acids with a weight content ranging from 0% to 30%, further optionally contains amino acids with a weight content ranging from 0% to 25%, further optionally contains amino acids with a weight content ranging from 0% to 20%, further optionally contains amino acids with a weight content ranging from 0% to 5%, further optionally contains amino acids with a weight content ranging from 3% to 25%, and further optionally contains amino acids with a weight content ranging from 5% to 20%),
and/or contains other components (optionally with a weight content ranging from 0% to 25%, further optionally with a weight content ranging from 0% to 20%, further optionally with a weight content ranging from 0% to 15%, further optionally with a weight content ranging from 0% to 11%, further optionally with a weight content ranging from 2% to 20%, and further optionally with a weight content ranging from 4% to 8%).

6. The use according to any one of claims 1 to 5, wherein: taking total weight percentage content of all components in the mulberry extract as 100%, the weight content of each component in the mulberry extract is as follows:
| | |
|---|---|
| alkaloids | 3% to 99%, |
| polysaccharides | 0.2% to 70%, |
| flavonoids | 0% to 10%, |
| amino acids | 0% to 50%, and |
| other components | 0% to 25%; |
optionally, taking total weight percentage content of all components in the mulberry extract as 100%, the weight content of each component in the mulberry extract is as follows:
| | |
|---|---|
| alkaloids | 15% to 99%, |
| polysaccharides | 0.2% to 20%, |
| flavonoids | 0% to 10%, |
| amino acids | 0% to 45%, and |
| other components | 0% to 15%; |
optionally, based on the mulberry extract, the weight content of each component is as follows:
| | |
|---|---|
| alkaloids | 15% to 80%, |
| polysaccharides | 5% to 35%, |
| flavonoids | 0% to 10%, |
| amino acids | 0% to 45%, and |
| other components | 0% to 15%; |
optionally, based on the mulberry extract, the weight content of each component is as follows:
| | |
|---|---|
| alkaloids | 15% to 70%, |
| polysaccharides | 0.2% to 20%, |
| flavonoids | 0% to 10%, |
| amino acids | 0% to 45%, and |
| other components | 0% to 15%; |
optionally, based on the mulberry extract, the weight content of each component is as follows:
| | |
|---|---|
| alkaloids | 15% to 70%, |
| polysaccharides | 0.2% to 20%, |
| flavonoids | 0% to 10%, |
| amino acids | 5% to 45%, and |
| other components | 0% to 15%; |
optionally, based on the mulberry extract, the weight content of each component is as follows:
| | |
|---|---|
| alkaloids | 15% to 65%, |
| polysaccharides | 0.2% to 20%, |
| flavonoids | 0% to 10%, |
| amino acids | 0% to 45%, and |
| other components | 0% to 15%; |
optionally, based on the mulberry extract, the weight content of each component is as follows:
| | |
|---|---|
| alkaloids | 15% to 65%, |
| polysaccharides | 0.2% to 20%, |
| flavonoids | 0% to 10%, |
| amino acids | 5% to 45%, and |
| other components | 0% to 15%; |
in the mulberry extract, the weight content of each component is as follows:
| | |
|---|---|
| alkaloids | 15% to 30%, |
| polysaccharides | 0.2% to 40%, |
| flavonoids | 0% to 10%, |
| amino acids | 20% to 50%, and |
| other components | 0% to 15%; |
optionally, taking total weight percentage content of all components in the mulberry extract as 100%, the weight content of each component in the mulberry extract is as follows:
| | |
|---|---|
| alkaloids | 30% to 99%, |
| polysaccharides | 0.2% to 35%, |
| flavonoids | 0% to 2%, |
| amino acids | 0% to 30%, and |
| other components | 0% to 20%; |
optionally, taking total weight percentage content of all components in the mulberry extract as 100%, the weight content of each component in the mulberry extract is as follows:
| | |
|---|---|
| alkaloids | 30% to 99%, |
| polysaccharides | 0.2% to 35%, |
| flavonoids | 0.05% to 2%, |
| amino acids | 0% to 25%, and |
| other components | 0% to 20%; |
optionally, taking total weight percentage content of all components in the mulberry extract as 100%, the weight content of each component in the mulberry extract is as follows:
| | |
|---|---|
| alkaloids | 40% to 99%, |
| polysaccharides | 0.2% to 25%, |
| flavonoids | 0.05% to 1%, |
| amino acids | 0% to 25%, and |
| other components | 0% to 15%; |
optionally, taking total weight percentage content of all components in the mulberry extract as 100%, the weight content of each component in the mulberry extract is as follows:
| | |
|---|---|
| alkaloids | 50% to 99%, |
| polysaccharides | 0.2% to 23%, |
| flavonoids | 0% to 1%, |
| amino acids | 0% to 20%, and |
| other components | 0% to 11%; |
optionally, taking total weight percentage content of all components in the mulberry extract as 100%, the weight content of each component in the mulberry extract is as follows:
| | |
|---|---|
| alkaloids | 50% to 99%, |
| polysaccharides | 0.2% to 35%, |
| flavonoids | 0% to 2%, |
| amino acids | 0% to 30%, and |
| other components | 0% to 20%; |
optionally, taking total weight percentage content of all components in the mulberry extract as 100%, the weight content of each component in the mulberry extract is as follows:
| | |
|---|---|
| alkaloids | 60% to 99%, |
| polysaccharides | 0.2% to 23%, |
| flavonoids | 0% to 1%, |
| amino acids | 0% to 5%, and |
| other components | 0% to 11%; |
further optionally, taking total weight percentage content of all components in the mulberry extract as 100%, the weight content of each component in the mulberry extract is as follows:
| | |
|---|---|
| alkaloids | 50% to 65%, |
| polysaccharides | 20% to 25%, |
| flavonoids | 0.5% to 1.5%, |
| amino acids | 3% to 25%, and |
| other components | 2% to 20%; and |
further optionally, taking total weight percentage content of all components in the mulberry extract as 100%, the weight content of each component in the mulberry extract is as follows:
| | |
|---|---|
| alkaloids | 50% to 65%, |
| polysaccharides | 20% to 25%, |
| flavonoids | 0.5% to 1.5%, |
| amino acids | 5% to 20%, and |
| other components | 4% to 8%; |
further preferably, the alkaloids include one or more of 1-deoxynojirimycin (DNJ), N-methyl-1-deoxynojirimycin, fagomine (FAG), 3-epi-fagomine, 1,4-dideoxy-1,4-imino-D-arabinitol (DAB), calysteginB2, calysteginC1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol; and
optionally, weight percentage of DNJ is no less than 50% (optionally 60% to 99%) of total alkaloids.

7. The use according to any one of claims 1 to 6, wherein:
the preparation of the mulberry extract includes the following steps of:
1) preparing a crude extraction solution of Moraceae plants; and
2) separating the crude extraction solution via a cation resin and/or an optional anion resin to afford a resin effluent, thereby affording the mulberry extract;
preferably, the method further includes the following steps of:
3) subjecting the resin effluent afforded in step 2) to alcohol precipitation and collecting supernatant; and
4) concentrating and/or drying the supernatant;
optionally, the method further includes the following steps of concentrating and/or drying the resin effluent afforded in step 2).

8. The use according to any one of claims 1 to 7, wherein: the mulberry extract acts on human beings or mammals.

9. The use according to any one of claims 1 to 8, wherein: the drug is in an oral dosage form.

10. The use according to any one of claims 1 to 9, wherein: the drug is prepared into tablets, capsules, oral solutions, oral emulsions, pills, granules, syrups and powder.
